# EUROPEAN PATENT SPECIFICATION

(11) **EP 3 917 418 B1**
(45) Date of publication and mention of the grant of the patent: **08.01.2025**
(21) Application number: 20708976.4
(22) Date of filing: 31.01.2020
(51) Int. Cl.: A61B 17/34

(54) **MEDICAL DELIVERY DEVICE FOR DELIVERING THERAPEUTIC AGENTS DIRECTLY INTO THE ANNULUS FIBROSUS OF AN INTERVERTEBRAL DISC**
MEDIZINISCHE ABGABEVORRICHTUNG ZUR DIREKTEN VERABREICHUNG THERAPEUTISCHER MITTEL IN DEN ANNULUS FIBROSUS EINER BANDSCHEIBE
DISPOSITIF D'ADMINISTRATION MÉDICALE D'AGENTS THÉRAPEUTIQUES DIRECTEMENT DANS L'ANNEAU FIBREUX D'UN DISQUE INTERVERTÉBRAL

(30) Priority: 01.02.2019 US 201962800215 P
(43) Date of publication of application: 08.12.2021
(73) Proprietor: Disccath, LLC, New York, NY 10128 (US)
(72) Inventor: LUTZ, Gregory, E., Pennington, NJ 08534 (US)
(74) Representative: AWA Sweden AB
(86) International application number: PCT/US2020/016167
(87) International publication number: WO 2020/160444

(56) References cited:
- WO-A1-02/19933
- WO-A2-01/56515
- US-A- 4 013 080
- US-A- 5 980 504
- US-A1- 2002 095 144
- US-A1- 2003 187 445
- US-A1- 2008 027 554
- US-A1- 2016 278 861

## Description

### RELATED APPLICATION

This application claims the benefit of U.S. Provisional Application No. 62/800,215, filed on February 1, 2019.

### BACKGROUND

### Description of Anatomy of the Disc of the Spine

The following background description has been adapted from col. 1, line 38 to col. 3, lines 36 of US 6,749,605 (Ashley et al.) and FIGS. 1A-1D are adapted from US 6,749,605.

Intervertebral disc abnormalities (e.g., morphologic) have a high incidence in the population and may result in pain and discomfort if internal tears develop and impinge on or irritate nerves. Disc abnormalities may be the result of trauma, repetitive use, metabolic disorders, and the aging process and include such disorders but are not limited to degenerative discs (i) localized tears or fissures in the annulus fibrosus, (ii) localized disc herniations with contained or escaped extrusions, and (iii) chronic, circumferential bulging disc.

Disc fissures occur rather easily after trauma or structural degeneration of fibrous components of the annulus fibrosus. Sneezing, bending or just attrition can tear these degenerated annulus fibers, creating a fissure. The fissure may or may not be accompanied by extrusion of nucleus pulposus material into or beyond the annulus fibrosus. The posterior annulus fibrosus is richly innervated and the contents of the nucleus pulposus are pro-inflammatory when they migrate into these tear, so damage to this region causes the majority of low back pain. The fissure itself may be the sole morphological change, above and beyond generalized degenerative changes in the connective tissue of the disc. Even if there is no visible extrusion, biochemicals within the disc may still irritate surrounding structures. Annular fissures can create severe pain and disability for patients and, because of a lack of blood supply, have a poor inherent capacity to heal. Initial treatment is symptomatic, including bed rest, pain killers and muscle relaxants. Spinal fusion with cages has been performed when conservative treatment did not relieve the pain. The fissure may also be associated with a herniation of that portion of the annulus.

With a contained disc herniation, there are no free nucleus fragments in the spinal canal. Nevertheless, even a contained disc herniation is problematic because the outward protrusion can press on the spinal nerves or irritate other structures. In addition to nerve root compression, escaped nucleus pulposus contents may chemically irritate neural structures. Current treatment methods include reduction of pressure on the annulus by removing some of the interior nucleus pulposus material by percutaneous nuclectomy. However, complications include disc space infection, nerve root injury, hematoma formation, instability of the adjacent vertebrae and collapse of the disc from decrease in height.

Another disc problem occurs when the disc bulges outward circumferentially in all directions and not just in one location. Over time, the disc weakens and takes on a "roll" shape or circumferential bulge. Mechanical stiffness of the joint is reduced, and the joint may become unstable. One vertebra may settle on top of another. This problem continues as the body ages, and accounts for shortened stature in old age. With the increasing life expectancy of the population, such degenerative disc disease and impairment of nerve function are becoming major public health problems. As the disc "roll" extends beyond the normal circumference, the disc height may be compromised, and foramina with nerve roots are compressed. In addition, osteophytes may form on the outer surface of the disc roll and further encroach on the spinal canal and foramina through which nerves pass. This condition is called lumbar spondylosis and spinal stenosis.

It has been thought that such disc degeneration creates segmental instability which disturbs sensitive structures which in turn register pain. Traditional, conservative methods of treatment include bed rest, pain medication, physical therapy or epidural steroid injections. Upon failure of conservative therapy, spinal pain (assumed to be due to instability) has been treated by spinal fusion, with or without instrumentation, which causes the vertebrae above and below the disc to grow solidly together and form a single, solid piece of bone. The procedure is carried out with or without discectomy. Other treatments include discectomy alone or disc decompression with or without fusion.

Nuclectomy can be performed by removing some of the nucleus to reduce pressure on the annulus. However, complications include disc space infection, nerve root injury, hematoma formation, and instability of adjacent vertebrae.

These interventions are problematic, because the alleviation of back pain is unpredictable even if surgery appears successful. In attempts to overcome these difficulties, fixation devices have been introduced to the market, including but not limited to pedicle screws and interbody fusion cages. Although pedicle screws provide a high fusion success rate, there is still no direct correlation between fusion success and patient improvement in function and pain. Studies on fusion have demonstrated success rates of between 50% and 67% for pain improvement, and a significant number of patients have more pain postoperatively. Therefore, alternative methods of improving the outcomes of patients with degenerative disc problems need to be explored, particularly through less invasive procedures.

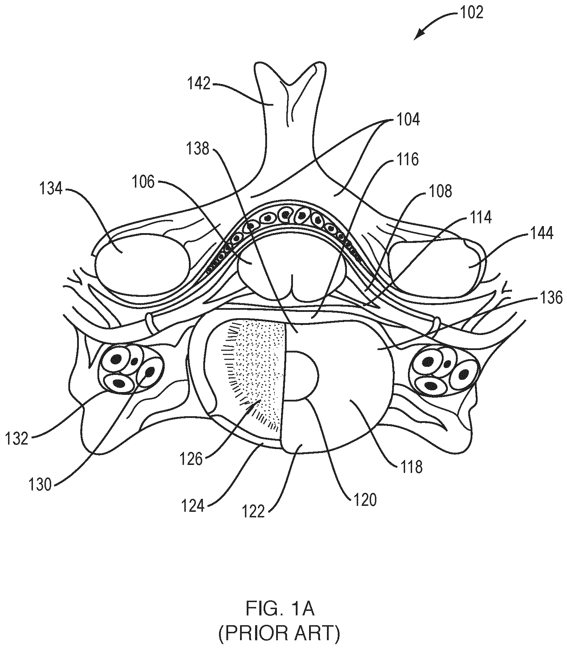

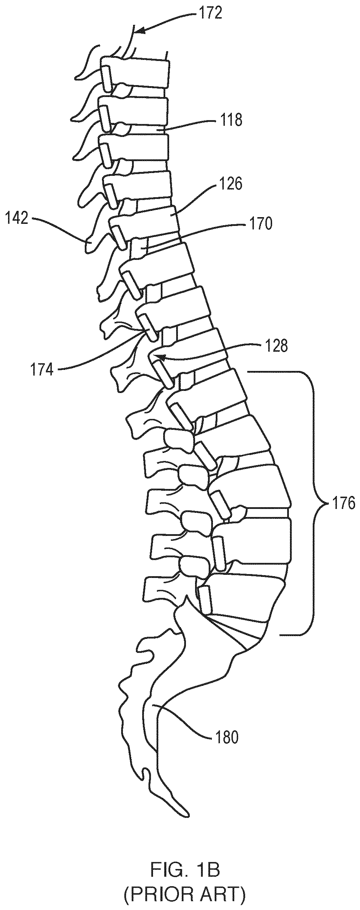

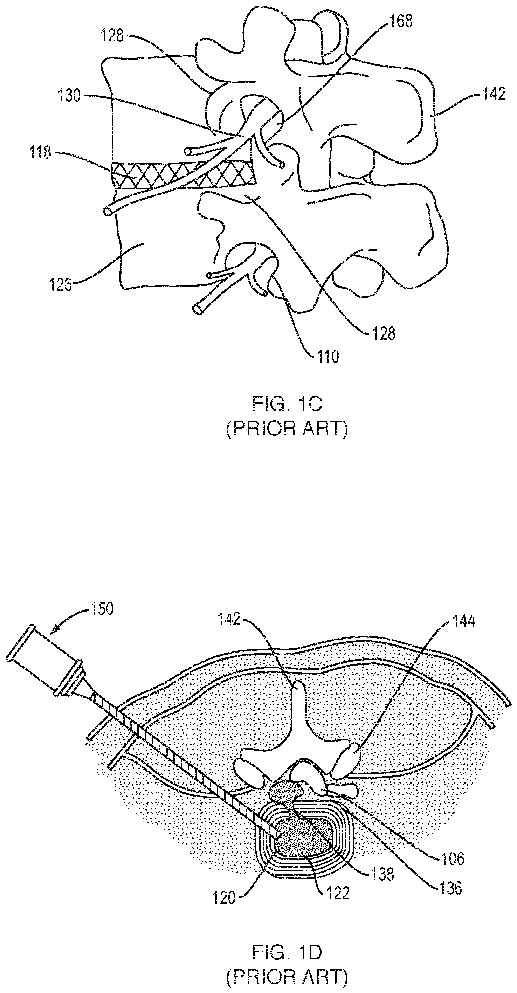

FIGS. 1A and 1B illustrate a cross-sectional anatomical view of a vertebra and associated disc and a lateral view of a portion of a lumbar and thoracic spine, respectively. Structures of a typical cervical vertebra (superior aspect) 102 are shown in FIG. 1A: 104-lamina; 106-spinal cord; 108-dorsal root of spinal nerve; 114-ventral root of spinal nerve; 116-posterior longitudinal ligament; 118-intervertebral disc; 120-nucleus pulposus; 122-annulus fibrosus; 124-anterior longitudinal ligament; 126-vertebral body; 128-pedicle (FIG. 1C); 130-vertebral artery; 132-vertebral veins; 134, 144-superior articular facets; 136-posterior lateral portion of the annulus; 138-posterior medial portion of the annulus; and 142-spinous process. In FIG. 1A, one side of the intervertebral disc 118 is not shown so that the anterior vertebral body 126 can be seen. FIG. 1B is a lateral aspect of the lower portion of a typical spinal column showing the entire lumbar region and part of the thoracic region and displaying the following structures: 118-intervertebral disc; 126-vertebral body; 142-spinous process; 170-inferior vertebral notch; 172-spinal nerve; 174-superior articular process; 176-lumbar curvature; and 180-sacrum.

FIG. 1C is a posterior-lateral anatomical view of two lumbar vertebrae. The presence of the spinal cord (nerve sac) and the posterior portion of the vertebral body 126, including the spinous process 142, and superior and inferior articular processes 110, prohibit introduction of a needle or trocar from a directly posterior position. This is important because the posterior wall of the disc 118 is the site of symptomatic annulus tears and disc protrusions/extrusions that compress or irritate spinal nerves for most degenerative disc syndromes. The inferior articular process, along with the pedicle 128 and the lumbar spinal nerve, form a small "triangular" window 168 through which introduction can be achieved from the posterior lateral approach.

FIG. 1D is a superior cross-sectional view of a cervical disc and vertebra with an introducer cannula positioned within the disc. FIG. 1D looks down on the instrument introduced by the posterior lateral approach. Percutaneous access to the disc is achieved by placing an introducer 150 into the disc from this posterior lateral approach, but the triangular window does not allow much room to maneuver. Once the introducer pierces the tough annulus fibrosus 122, the introducer is fixed at two points along its length and has very little freedom of movement. Thus, this approach has allowed access only to small central and anterior portions of the nucleus pulposus 120. Current methods do not permit percutaneous access to the posterior half of the nucleus or to the posterior wall of the disc (e.g., 136, 138). Major and potentially dangerous surgery is required to access these areas.
US5980504 discloses an externally guidable intervertebral disc apparatus manipulates disc tissue present at a selected location of an intervertebral disc, comprising a catheter having a distal end, a proximal end, and a longitudinal axis, the catheter having an intradiscal section at the distal end of the catheter, the intradiscal section being extendible into the disc, having sufficient rigidity to be advanceable through the nucleus pulposus of the disc under a force applied longitudinally to the proximal end, having sufficient flexibility in a direction of the disc plane to be compliant with the inner wall, having insufficient penetration ability to be advanceable through the inner wall of the annulus fibrosus under the applied force; and a functional element located in the intradiscal section for adding or removing energy or a material at the selected location of the disc.
WO0219933A1 discloses a system and method for approaching the intervertebral disc through a percutaneous insertion from the of a patient for thermal or electromagnetic treatment of an intervertebral disc, includes an elongated probe member having a guidable region adjacent its distal end with an undulating groove defined in its outer region in at least one radial direction of movement relative to a longitudinal axis of the thermal prove. The guidable region in WO0219933A1 includes a plurality of undulating grooves, whereby adjacent undulating grooves are longitudinally spaced with respect to each other. The undulating grooves each define a sinusoidal configuration which may be arranged about an undulating axis extending in oblique relation to the longitudinal axis. The guidable region also includes a longitudinally extending backbone may also include a cannula to facilitate introduction of the thermal probe in the intervertebral disc. The cannula includes an arcuate end portion dimensions to arranged the guidable region of the thermal probe at a desired orientation within the annulus fibrosis.
US2002095144A1 relates to devices and methods for altering the tissue in and around an intervertebral disc through localized hypothermia therapy to restore function of the disc and reduce pain.
US2016278861A1 relates to treatment method using multiple energy sources. The treatment method using multiple energy sources, includes: applying a first energy into an inside of an annulus fibrosus; and applying a second energy different from the first energy to an outside of the annulus fibrosus.
US2003187445A1 discloses devices and methods for treating a damaged intervertebral disc to reduce or eliminate associated back pain. US2003187445A1 provides disc reinforcement therapy (DRT) which involves implanting one or more reinforcement members in and around the annulus of the disc. The reinforcement members may be used to stabilize the annulus and/or compresses a portion of the annulus so as to reduce a bulge and/or close a fissure. The implantable devices and associated delivery tools may incorporate heating capabilities to thermally treat the annular tissue. Alternatively or in combination, other devices may be specifically employed for such thermal treatment.

### SUMMARY

It is desirable to diagnose and treat disc abnormalities such as disc degeneration at locations previously not accessible or identifiable via percutaneous approaches and without major surgical intervention or substantial destruction to the disc.

The invention is defined by the appended independent claim, with embodiments set forth in the appended dependent claims, in the following description and in the drawings.

Embodiments provide a minimally invasive apparatus for diagnosing and treating fissures of discs around substantially the entire circumference of the annulus fibrosus of the disc. The cells in the annulus fibrosus of the disc respond well to biologic therapy to stimulate repair. The more precisely cells can be delivered to the damaged areas the better the anticipated clinical outcomes.

Embodiments provide a device which has a distal end that is inserted into the annulus fibrosus of an intervertebral disc and accesses the posterior, posterior lateral and the posterior medial regions between the inner and outer wall of the annulus fibrosus for delivery of a biologic therapy.

Embodiments provide an apparatus which is advanceable and navigable through tissue of and around the inner wall of the annulus fibrosus to provide a geographic biologic treatment to multiple identified and unidentified annular fissures.

Embodiments provide a non-destructive apparatus for treating morphologic abnormalities of discs.

Embodiments provide an apparatus to treat degenerative intervertebral discs by delivering a biologic to repair selective nerves embedded in the walls of the annulus fibrosus.

Embodiments provide an apparatus to treat degenerative intervertebral discs which is advanceable and navigational between the inner and outer wall of the annulus fibrosus without entry into the nucleus pulposus.

Embodiments of the present invention are designed to stay within the walls of the annulus fibrosus. Embodiments can include a closed end blunt tip or cap, that can be offset, with a side port that is designed to be orientated within the annulus to direct fluid flow during injection from the peripheral annulus toward the center nucleus pulposus. Embodiments can be used to deliver a biologic or, more specifically, an autologous biologic, such as marrow or Platelet Rich Plasma (PRP). When delivering a biologic to tissue, one would not typically heat the delivery catheter or cannula or deliver heat to the tissue, as heat can damage the biologic or render it ineffective. In some embodiments, a removable stylet is provided for placement within the delivery catheter. The removable stylet can be centerless ground to adjust its diameter over its effective length, can also have a pre-set bend, and can be made from a shape memory material such as nitinol. The stylet is removed after the delivery catheter is inserted to the desired position to allow for the injection of the biologic into the disc.

Damage to the annulus fibrosus is a result of a local weakening of the annular fibers. However, it is not possible to visualize the annulus architecture definitively prior to the procedure, and this architecture varies from patient to patient. Therefore, certain embodiments of this device include design elements allowing the stylet and/or catheter the structural flexibility to adapt to the internal tissue integrity of the annulus in a dynamic manner. This flexibility can be achieved through a combination of material choice and stiffness, a preset arc at the tip and potentially along the length of the device, or centerless grinding of the stylet. The resulting flexibility will allow the device to traverse the annulus circumferentially along the path of least resistance (i.e. the path with weaker annular tissue), ultimately allowing for the precise placement of the tip in the area of greatest tissue damage, thereby delivering the therapeutic agent broadly throughout the whole posterior annulus fibrosus.

A device for delivering a therapeutic agent percutaneously to a target tissue of an intervertebral disc includes an introducer needle and a delivery catheter. The introducer needle can be rigid and includes an elongated cannula having a proximal end and a distal end, the proximal and distal ends forming openings and being in communication along the cannula. A stylet extends through the cannula and has a tip that closes the opening at the distal end of the cannula. The cannula and stylet are configured to allow insertion of the needle percutaneously into tissue. The delivery catheter fits coaxially through the introducer needle when the stylet is removed, is longer than the introducer needle and includes an elongated hollow shaft that has a preset bend providing a pre-shaped curved portion along a length of the delivery catheter. The delivery catheter has a proximal end and a distal end, the proximal and distal ends forming openings and being in communication along the hollow shaft. The distal opening is a side opening to the inside of the preset bend. The distal end of the delivery catheter includes a closed, blunt tip. The delivery catheter is extendable from the distal end of the introducer needle and into a target tissue of an intervertebral disc, the combination of the blunt tip and preset bend allowing the delivery catheter to advance through but not out of an annulus fibrosus of the intervertebral disc. The side opening is orientable toward a center of the intervertebral disc for delivering a therapeutic agent to the target tissue.

The blunt tip can be positioned on the shaft at the distal end of the delivery catheter at an angle that is offset from the center line of the delivery catheter. The blunt tip can be welded on the hollow shaft at the distal end of the delivery catheter and made of a material different from the shaft. For example, the hollow shaft of the delivery catheter can be made of nitinol. The blunt tip of the delivery catheter can be made of stainless steel.

The cannula of the introducer needle can have a preset bend at the distal end of the cannula. The preset bend at the distal end of the cannula can be about 3 mm to about 9 mm, preferably about 6.25mm, from the distal tip with an angle between about 4 degrees and about 6 degrees, preferably with an angle of about 5 degrees.

The introducer needle can include a two-part handle with a first part of the handle attached to the cannula and a second part of the handle attached to the stylet, each part attached at the proximal end of the introducer needle. The second part of the handle and stylet can be selectively removable from the cannula, and the first part of the handle can define a passageway in communication with the opening at the distal end of the introducer needle when the first part of the handle and stylet are removed. The first part of the handle can include a luer hub.

The delivery catheter can include a luer lock connection at the proximal end of the delivery catheter. The luer lock enables delivery of the therapeutic agent from a syringe holding the therapeutic agent and being coupled to the luer lock.

The introducer needle and delivery catheter can have markings on the outside that allow determining the distance of insertion into the target tissue. For example, the delivery catheter can have one or more markings on the proximal end on the outside that allow determining the distance the delivery catheter is inserted beyond the introducer needle or retracted from the target tissue.

The target tissue can be a posterior outer third of the annulus fibrosus of the intervertebral disc.

The introducer needle can be configured to be inserted a pre-determined depth into the annulus fibrosus so that the combination of the starting point where the distal end of the delivery catheter exits the distal end of the introducer needle, the length the delivery catheter extends beyond the introducer needle when the catheter is fully extended beyond the distal end of the introducer needle, the preset bend of the delivery catheter, the blunt tip on the end of the delivery catheter, and stiffness or persistence length of the delivery catheter, results in the delivery catheter exiting into and staying within the tissue of the annulus fibrosus.

The delivery catheter can be configured to be retracted from the target tissue in set intervals, at each interval, a portion of a therapeutic agent being introduced into the annulus fibrosus. The therapeutic agent can be platelet rich plasma, bone marrow aspirate, bone marrow concentrate, adipose-derived cells, autologous or non-autologous fibrin, medications, contrast dye, or energy delivered over a fiber or probe.

The preset bend of delivery catheter is such that the catheter upon exiting the introducer needle follows an oval path having an initial diameter between about 1.5 cm and about 3.5 cm.

The length that the delivery catheter is extendable past the distal end of the introducer needle can be sufficient to travel at least 75% of the circumference of a circle based on the preset bend of the delivery catheter.

A method not according to the claimed invention for minimally invasive delivery of a therapeutic agent percutaneously to target tissue of an intervertebral disc includes advancing a delivery catheter into target tissue of an intervertebral disc through an introducer needle placed in the intervertebral disc. The delivery catheter is longer than the introducer needle and has a proximal end and a distal end, the proximal and distal ends forming openings and being in communication along the delivery catheter. The distal opening of the catheter is a side opening. The distal end of the delivery catheter includes a blunt tip such that when the distal end of the delivery catheter is extended from the distal end of the introducer needle, the blunt tip allows the delivery catheter to advance through but not out of an annulus fibrosus of the intervertebral disc. The method further includes delivering a therapeutic agent through the distal side opening to the target tissue.

The delivery catheter can have a preset bend and the side opening can be to the inside of the preset bend. The location of the side opening facilitates delivery of the therapeutic agent to the target tissue, which can be a posterior outer third of the annulus fibrosus of the intervertebral disc.

Delivering the therapeutic agent can include retracting the delivery catheter from the target tissue in set intervals and, at each interval, delivering a portion of the therapeutic agent to the target tissue. The therapeutic agent being delivered can include platelet rich plasma, bone marrow aspirate, bone marrow concentrate, adipose-derived cells, autologous or non-autologous fibrin, medications, contrast dye, or energy delivered over a fiber or probe.

The method can further include inserting the introducer needle with a stylet positioned coaxially within the introducer needle to a pre-determined depth into the annulus fibrosus, and removing the stylet prior to advancing the delivery catheter. Advancing the delivery catheter into the target tissue can include guiding the delivery catheter with a blunt stylet positioned coaxially within the delivery catheter.

### BRIEF DESCRIPTION OF THE DRAWINGS

The foregoing will be apparent from the following more particular description of example embodiments, as illustrated in the accompanying drawings in which like reference characters refer to the same parts throughout the different views. The drawings are not necessarily to scale, emphasis instead being placed upon illustrating embodiments.
FIG. 1A is a superior cross-sectional anatomical view of a cervical disc and vertebra.
FIG. 1B is a lateral anatomical view of a portion of a lumbar and thoracic spine.
FIG. 1C is a posterior-lateral anatomical view of two lumbar vertebrae.
FIG. 1D is a superior cross-sectional view of a cervical disc and vertebra with an introducer cannula positioned within the disc.
FIG. 2A is a plan view of an introducer needle, with sharp stylet and luer connection at the hub.
FIG. 2B is a plan view of an introducer needle, with stylet, luer connection at the hub and with a pre-set bend on the distal end of the needle. The angle of the bend in the introducer needle is determined in relation to the bend of the delivery catheter so that the combination of the delivery catheter that fits coaxially through the introducer needle allows the delivery catheter to stay within the tissue of the annulus fibrosus and thus does not enter the center nucleus pulposus or surrounding tissue.
FIG. 3A is a plan view of a delivery catheter. The combination of adjusting the depth of the introducer needle and the pre-set bend of the delivery catheter will allow the curved delivery catheter to traverse a large geography of the annular fibrosus of the disc without exiting into the nucleus pulposus or exiting outside the annular fibrosus.
FIG. 3B is a detail view of a distal, curved portion of the delivery catheter of FIG. 3A in one contemplated embodiment.
FIG. 3C is a detail view of the closed blunt end tip of the delivery cannula of FIG. 3B. The blunt tip is welded onto the end of a hollow shaft of the delivery catheter at an angle and has a side port.
FIG. 4A illustrates the introducer needle and the delivery catheter deployed coaxially through the introducer needle. The angle of the bend in the delivery cannula and closed blunt tip that fits coaxially through the introducer needle allows the delivery catheter to stay within the tissue of the annulus fibrosus and thus does not enter the center nucleus pulposus or surrounding tissue.
FIG. 4B is a view of the introducer needle positioned in the disc and the delivery catheter deployed coaxially through the introducer needle. The delivery catheter has a pre-set bend such that the delivery catheter stays within the tissue of the annulus fibrosus.
FIG. 5 illustrates an introducer needle.
FIGS. 6A and 6B are top and side views, respectively, of an introducer stylet.
FIG. 7A illustrates the introducer needle of FIG. 5 assembled with the stylet of FIG. 6A.
FIG. 7B is a side view of the introducer needle and stylet of FIG. 7A illustrating a bend formed in the distal tip portion.
FIG. 7C illustrates the introducer needle and stylet of FIG. 7B with the distal tip portion shortened and provided with a sharp, beveled tip.
FIG. 7D is a side view of the introducer needle and stylet of FIG. 7C.
FIG. 7E is detail view of the distal tip of the assembled introducer needle and stylet of FIG. 7D illustrating the distal tip of the stylet closing the distal opening in the cannula of the introducer needle.
FIGS. 8A-8C are side, top, and front views, respectively, of a delivery catheter.
FIG. 9 illustrates the delivery catheter of FIG. 8A extended through the introducer needle of FIG. 7C after the stylet has been removed.

### DETAILED DESCRIPTION

A description of example embodiments follows.

The terms "delivery catheter" and phrase "cannula of the delivery needle" are used in this disclosure to describe the hollow, longer catheter that fits coaxially through the shorter introducer needle.

An oval (from Latin ovum, "egg") is a closed curve in a plane which "loosely" resembles the outline of an egg. Many distinct curves are commonly called ovals or are said to have an "oval shape." Generally, to be called an oval, a plane curve should resemble the outline of an egg or an ellipse. In particular, these are common traits of ovals: 1. they are differentiable (smooth-looking), simple (not self-intersecting), convex, closed, plane curves; 2. their shape does not depart much from that of an ellipse, and 3. an oval would generally have an axis of symmetry, but this is not required. (Source: Oval, Wikipedia,
https://en.wikipedia.org/wiki/Oval, accessed January 30, 2019.)

In mathematics, an ellipse is a curve in a plane surrounding two focal points such that the sum of the distances to the two focal points is constant for every point on the curve. As such, it is a generalization of a circle, which is a special type of an ellipse having both focal points at the same location. (Source: Ellipse, Wikipedia,
https://en.wikipedia.org/wiki/Ellipse, accessed January 30, 2019.)

The persistence length is a basic mechanical property quantifying the stiffness of a polymer. (Source: Persistence length, Wikipedia,
https://en.wikipedia.org/wiki/Persistence_length, accessed January 30, 2019.)

The intervertebral disc is composed of a gelatinous central core (the nucleus pulposus) which is contained by 10-20 circumferentially oriented collagen fiber rings (the annulus fibrosus). There is a transition zone from the nucleus pulposus to the annulus fibrosus, whereby the outer fibers of the annulus fibrosus are generally stronger than the inner fibers.

Centerless grinding is a term that refers to selectively removing material from a solid wire to have a variable outer diameter (OD) along its length.

### Description of Prior Approaches

There are numerous prior approaches that use an introducer needle with a stylet to penetrate through the annulus fibrosus. The introducer needle when the stylet is removed creates a working channel to introduce a second longer needle or needles or probe into the nucleus pulposus. These double needle systems generally are either straight, curved, or a combination of straight and curved. These systems are designed to access a particular targeted treatment zone or multiple zones. Some of the approaches allow the shape of the combined channel created by the assembled cannulas of the device to access the target zone by traversing through both sides of the annulus fibrosis, across the nucleus pulposus, and through the inner wall of the annulus fibrosus distal to where the first puncture was made. Some approaches use curved needles.

For example, US 9,113,950 to Schultz et al. teaches a therapeutic delivery device and associated method that includes a needle assembly. According to Schultz, the needle assembly can include either: 1) an introducer needle that internally constrains an advanceable cannula or 2) an introducer needle having the advanceable cannula and further includes an advanceable catheter internally constrained within the cannula. Schultz describes aspects of the needle assembly allow the cannula and catheter to be independently advanced by a user from the introducer needed to exact distances and with exact arch.

Some of the known approaches deliver therapy inside the nucleus pulposus by targeting the inner wall of the annulus fibrosus through a curved probe that conforms to the curved shape of the nucleus pulposus. Energy in the form of heat can be delivered over a second cannula or probe introduced into the nucleus pulposus through a first cannula. The heat can be sufficient to coagulate tissue as a means to seal the disc. The probe used to deliver the energy can be delivered over a guide wire that is introduced through a cannula of an introducer needle. See, for example, US 6749605B2 to Ashley et al., US 6,007,570 to Sharkey et al., and US 6,878,155 to Sharkey et al.

Some art describes the use of needles to transverse the pedicle bone to access the marrow space of the vertebral body. See, for example, Pakter et al. 8,747,359, McGuckin et al. 8,784,382. Cook Medical sells a device called the Pakter needle system comprised of two needles, one introducer needle and a second delivery needle. The second delivery needle has a pre-set bend, is made of nitinol, fits coaxially through the introducer needle, and bends to access the inside of the nucleus pulposus.

Shah et al. describe a double needle system to deliver narcotic to nerves in the spine following failed back surgery. (Shah et al. Targeting the spinal nerve via a double-needle, transforaminal approach in failed back surgery syndrome: Demonstration of a technique. Pain physician. 7. 93-7. Feb 2004)

Kumar et al. describe a double needle technique to access the nucleus pulposus. (Kumar, Naresh & Agorastides, Ioannis. (2000). The curved needle technique for accessing the L5/S1 disc space. The British journal of radiology. 73. 655-7.
10.1259/bjr.73.870.10911790.)

However, painful tears and herniations occur across and around the posterior circumference of the disc of the spine. These tears are not always easily identified, can be diffuse, and do not always communicate with the inner nucleus pulposus. Therefore, therapeutic agents delivered through entry of the nucleus pulposus do not necessarily flow throughout all of the painful tears. Prior needle systems are typically designed to access an individual zone or zones and often miss entire areas of damage, thereby limiting the potential therapeutic benefits of the injectate. In addition, the blood supply of the intervertebral disc is limited to the outer third of the annulus fibrosus. Delivery of fluids, e.g., biologic agents, to stimulate perivascular repair cells needs to be as close to the posterior annulus as possible.

There currently exists no device to reliably deliver therapeutic agents precisely throughout the entire posterior annulus fibrosus. Therefore, a better means of delivering therapy to the entire posterior circumference of the disc of the spine is needed. It will be appreciated that a device with the features described herein can also be used for the delivery of other materials (i.e. contrast agent or anesthetics) useful in the diagnosis and/or treatment of annular tears and fissures.

### Example Embodiments

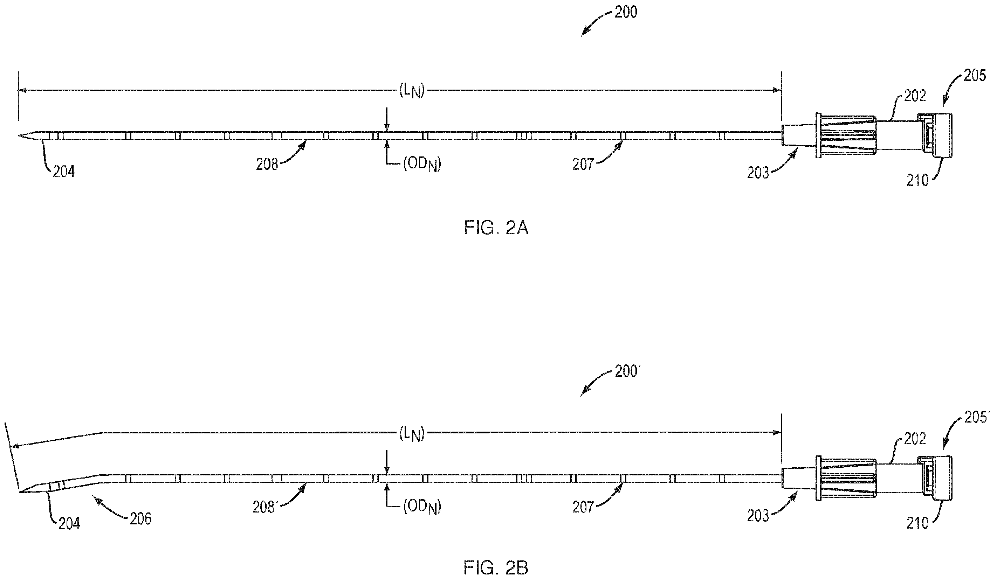

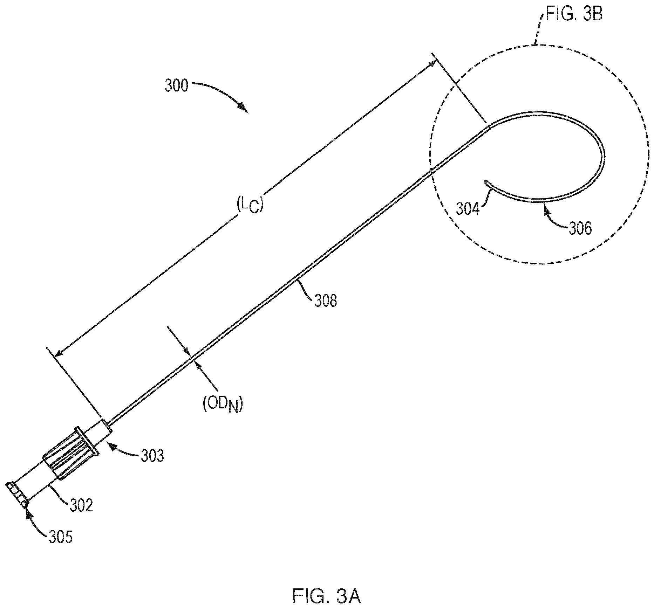

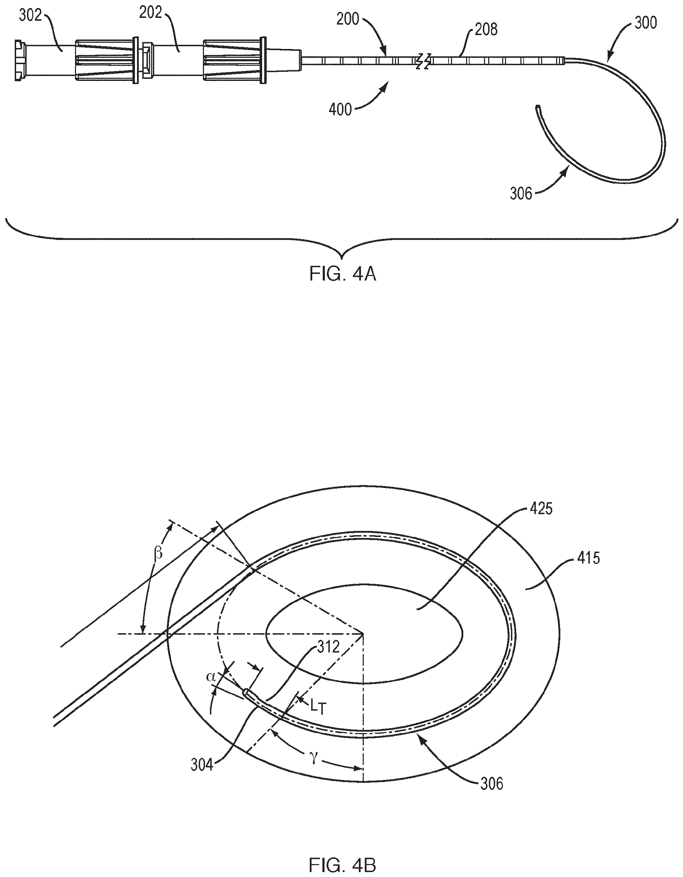

FIGS. 2A to 4B illustrate example embodiments of a device for delivering a therapeutic agent into the intervertebral disc includes an insertion or introducer needle (see FIGS. 2A and 2B) with a removable stylet and a delivery catheter/cannula (see FIGS. 3A-3C, 4A, and 4B) that is receivable in the insertion needle and has a pre-shaped curve/bend and a blunt tip.

FIG. 2A shows an introducer needle 200, with an elongated cannula 208, a distal end 204, and a luer connection at a hub 202 at the proximal end 203 of the needle 200. A sharp stylet 205 extends through the hub 202 and elongated cannula 208. The cap 210 at the proximal end of the stylet is visible in FIG. 2A. The cap 210 and hub 202 form a two-part handle of the assembly introducer needle 200.

FIG. 2B shows an introducer needle 200', with a stylet 205', luer connection at a hub 202 and with a pre-set bend 206 on a distal end 204 of the needle 200'. Only the cap 210 of the stylet 205' is visible in FIG. 2B. The cap 210 and hub 202 form a two-part handle of the assembly introducer needle 200'. The pre-set bend 206 at the distal end of the needle can be about 3 mm to about 9 mm, preferably about 5 mm to about 7 mm, more preferably about 6.25 mm, from the distal tip with an angle between about 2 degrees and about 10 degrees, preferably between about 4 degrees and about 7 degrees, and more preferably with an angle of about 5 degrees. The distal end 204 of the cannula has a sharp, beveled tip to facilitate penetration into tissue.

As illustrated in FIGS. 2A and 2B, the introducer needles 200, 200' can have markings 207 on the outside that allow determining the distance of insertion into the target tissue. The cannula 208, 208' has an exposed length L_{N} and an outer diameter OD_{N}. In example embodiments, L_{N} is about 6.0 inches (152.4 mm) and OD_{N} is about 0.058 inches (1.473 mm) corresponding to an outer diameter of a 17-gauge tube.

FIG. 3A illustrates a delivery catheter 300 that includes a luer connection 305 (e.g., a luer lock) at hub 302 at a proximal end 303, a tip 304 at a distal end, and an elongated hollow shaft 308. The catheter has a pre-shaped curve/bend 306 and a blunt tip portion 301 at tip 304. The catheter 300 can be formed from Nitinol, although the tip portion 304 can be steel welded to a Nitinol portion, e.g., the hollow shaft 308. The catheter can have markings, which may be similar to the markings of the introducer cannula as illustrated in FIG. 2A and 2B. An exposed length Lc of the hollow shaft 308 of the catheter can be about 5.0 inches (127.0 mm) and an outer diameter ODc can be about 0.032 inches (0.813 mm) corresponding to an outer diameter of a 21-gauge tube.

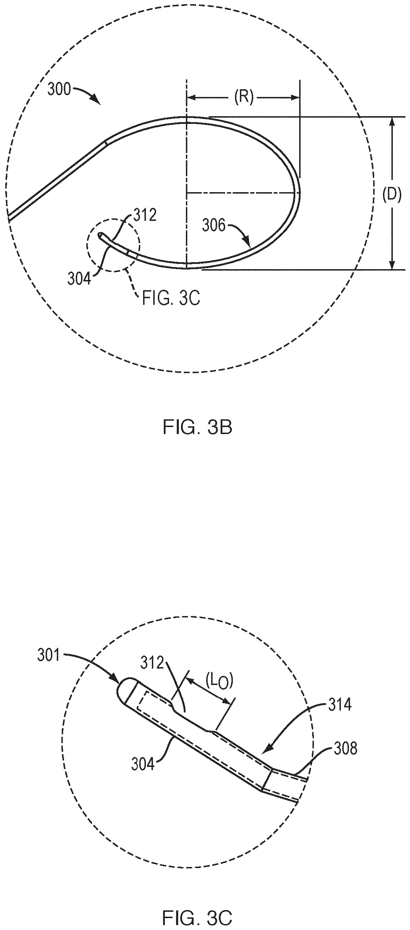

FIG. 3B is a detail view of a distal, curved portion 306 of the delivery catheter 300 of FIG. 3A in one contemplated embodiment. The curved portion, e.g., preset bend, of delivery catheter 300 is such that the catheter, upon exiting the introducer needle, follows an oval path having an initial diameter between about 15 mm and about 35 mm. The bend portion 306 can be characterized by a radius R and diameter D, as illustrated. In an embodiment, R is about 0.678 inches (17.2 mm) and D is about 0.918 inches (23.3 mm).

As illustrated in FIGS. 3B, 3C and 4B, the catheter 300 includes distal side opening (side port) 312. As shown, the opening 312 is to the inside of the preset bend 306. The side opening may be laser cut into the distal tip 304. As illustrated in FIG. 3C, the opening is elongated, having a length Lo along a longitudinal axis of the catheter 300. The length of the distal tip 304 is L_{T}, as illustrated in FIG. 4B. In an embodiment, Lo is about 0.075 inches (1.9 mm) and L_{T} is about 0.20 inches (5.1 mm). In addition to the pre-set bend 306 of the catheter that provides the distal curve shaped portion, the tip portion 304 can include an offset angle or bend 314, best illustrated in FIGS. 3C and 4B. The bend 314 at the distal end of the catheter 300 can be about 3 mm to about 6 mm from the distal tip 301 with an angle α between about 5 degrees and about 45 degrees, preferably with an angle of about 10 degrees. As illustrated, the blunt tip portion 301 can be rounded and form a closed distal end of the catheter 300.

FIG. 4A illustrates the device 400 including the introducer needle 200 and the delivery catheter 300 deployed coaxially through the introducer needle 200. The delivery catheter 300 is longer than the introducer needle 200, such that at least a portion of the delivery catheter, e.g., the curved portion 306, is extendable beyond the distal end of the introducer needle.

In use, the introducer needle 200 is inserted with the stylet 205 through the skin and into the annulus fibrosus of the intervertebral disc. The annulus is the fibrous outer tissue that surrounds the softer nucleus of the disc. The stylet 205 is removed and the catheter 300 is inserted through the introducer needle 200 into the annulus. Unlike prior approaches, the catheter 300 does not enter the nucleus but stays within the annulus. FIG. 4B schematically illustrates the annulus 415, bounded by dashed, elliptical curves and surrounding the nucleus 425. Because of the pre-set shape 306 and the blunt tip 304, the catheter 300 follows the ring-like fibrous structure of the annulus 415 and stays within the tissue of the annulus. The catheter does not enter surrounding tissues or the nucleus 425 of the disc. The therapeutic agent is delivered into annulus tissue as the catheter 300 is retracted incrementally through the annulus. Delivery is through the side port 312 near the distal end of the catheter 300. A preferred target site is the posterior outer annulus fibrosus of the lumbar intervertebral disc. The preset bend 306 can have multiple angles of curvature and the catheter 300 follows an oval shape, as illustrated in FIG. 4B. The curve of the catheter starts at a horizontal angle β and extends past 180° vertical to a vertical angel γ. In an embodiment, β=30° and γ=45° for a total angle of curvature of 285°.

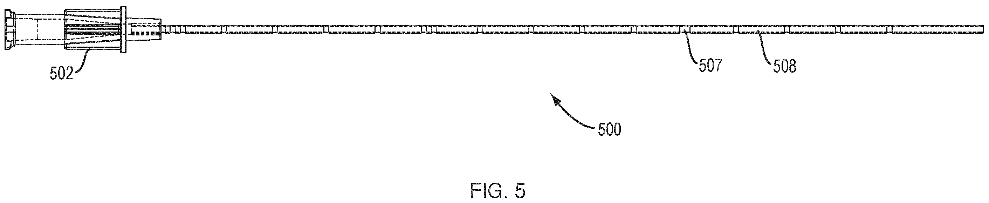

FIGS. 5-9 illustrate components of a minimally invasive device for delivering a therapeutic agent percutaneously to a target tissue of an intervertebral disc, the device having features similar to those described above with reference to FIGS. 2A-4B.

FIG. 5 illustrates an introducer needle 500 with a hub 502 at a proximal end and an elongated cannula 508 extending from the proximal end. The hub is a luer hub that includes a luer connector. The elongated cannula is provided with marking 507 that facilitate monitoring of depth of entry of the cannula into tissue. The distal end of the cannula 508 is blunt and unfinished. As described below, the distal end is shorted and sharpened, which is can be done with the introduce style in place.

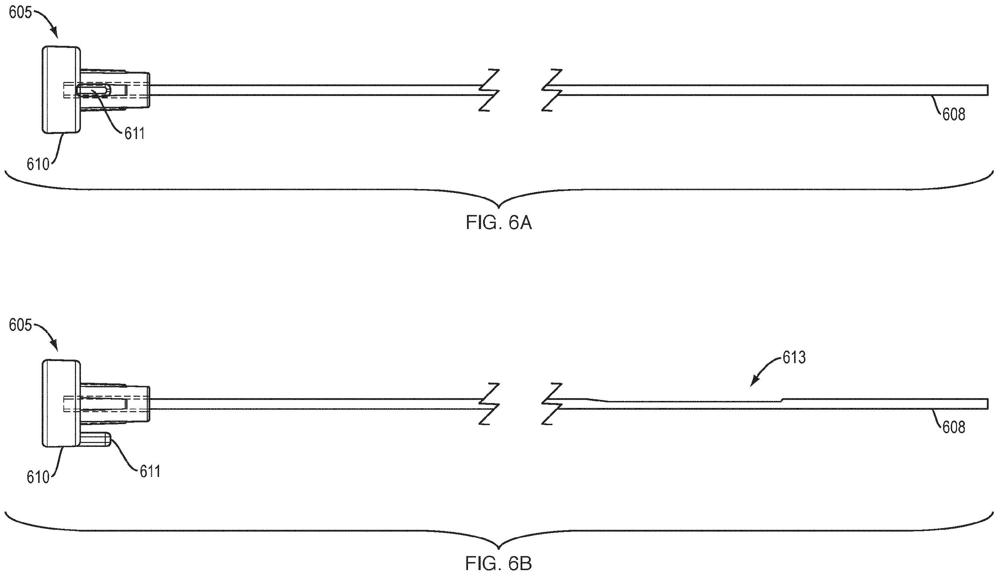

FIGS. 6A and 6B illustrate an introducer stylet 605, which is configured for assembly with the introducer needle of FIG. 5. The stylet 605 includes a handle 610 at a proximal end and an elongated, solid shaft 608. The shaft can be formed from stainless steel wire. The outer diameter of the shaft 608 is smaller than an inner diameter of the cannula 508 of the introducer needle, such that the shaft 608 can be extended through the cannula 508. As tapered portion of the handle 610 is configured to fit the luer connector of the hub 502 of the introducer needle. The handle 610 includes a marker 611 that allows the operator to orient the stylet and introducer needle once inserted into tissue, as further described below. As shown, the marker 611 is a pin that extends distally from the handle of the stylet. The pin can be configured to fit a notch formed in the handle or hub of the introducer needle 500. As illustrated in FIG. 6B, the shaft 608 can have a reduced diameter profile 613 along a length of the shaft. The profile 613 may be positioned near but spaced from the distal end of the shaft.

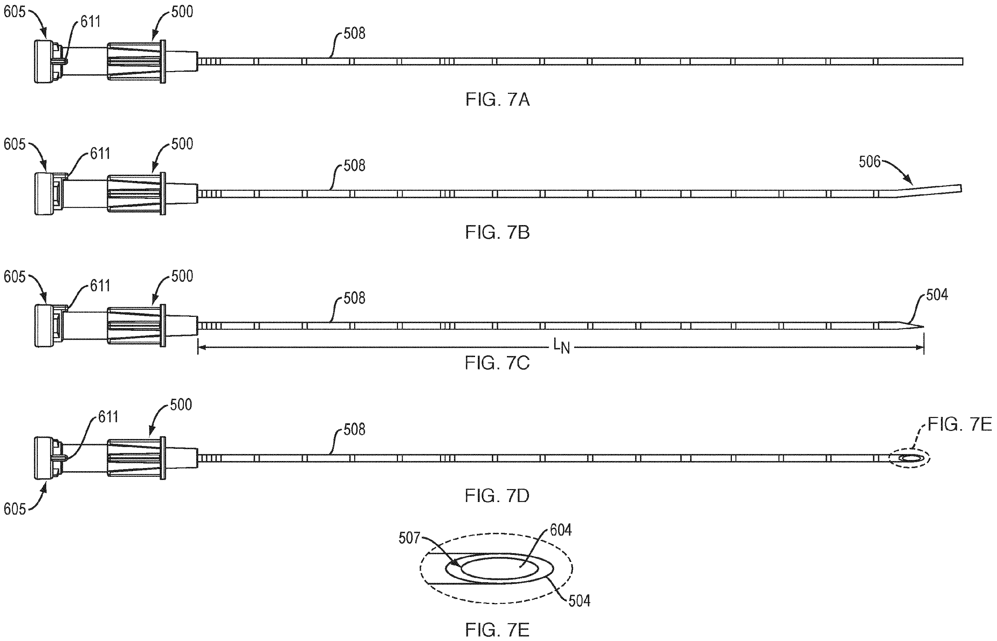

FIG. 7A illustrates the introducer needle 500 of FIG. 5 assembled with the stylet 605 of FIG. 6A. The shaft of the stylet 605 is ended through the elongated cannula 508 of the introducer needle. The tapered portion of handle 610 of the stylet is coupled to the hub 502, with the pin 611 positioned in a notch of the hub 502. Once the introducer needle and stylet are assembled, a bend 506 can be formed in the distal tip portion as illustrated in FIG. 7B. The angle of the bend can be chosen based on the application and target tissue. FIG. 7B shows a bend that forms an angle of about 5° with the major longitudinal axis of the cannula 508. Such an angled has been found useful for accessing an intervertebral disc.

As illustrated in FIGS. 7C and 7D, the assembled introducer needle 500 and stylet 605 can be further processed to shorten the distal tip portion and form a sharp, beveled tip, e.g., by grinding or other suitable means. FIG. 7E is detail view of the distal tip of the assembled introducer needle and stylet illustrating the distal tip 604 of the shaft of the stylet 605 closing the opening 507 at the distal end 504 of the cannula 508 of the introducer needle. It is desirable that the distal tip 604 of the stylet is flush with the bevel of the cannula 508, to avoid snagging or tearing as the device is inserted into tissue. The bevel and opening at the distal end of the cannula 508 are aligned with the pin 611 at the handle of the stylet 605. This allows the operator to orient the bevel and opening once the introducer needle is inserted into tissue. Preferably, the bevel and opening face the inside of the vertebral disc. In this way, when the stylet is removed and a delivery catheter is inserted through the introducer cannula, the delivery catheter exits the introducer cannula toward the inside of the disc.

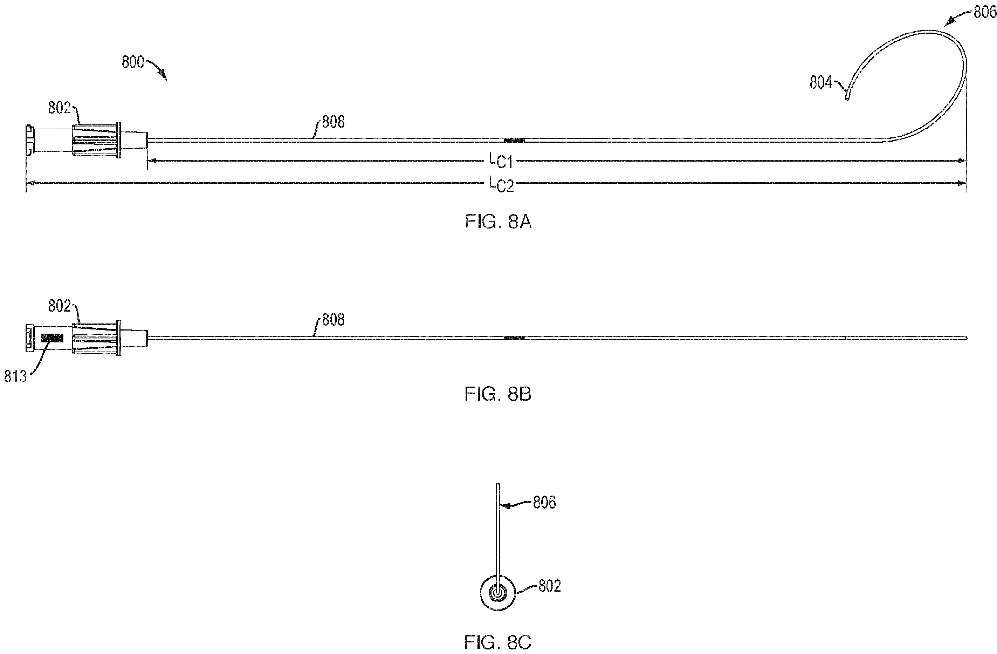

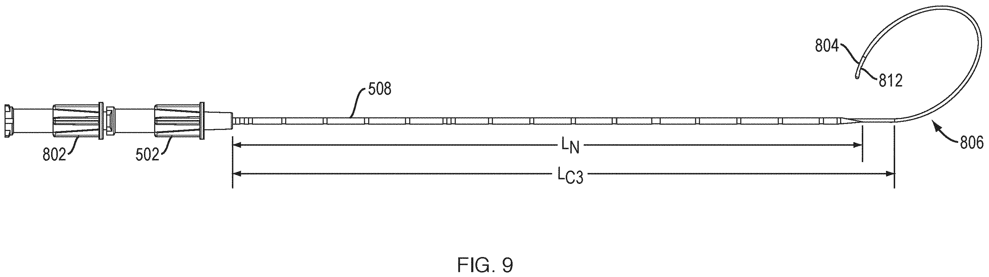

FIGS. 8A-8C illustrate a delivery catheter 800 configured to be inserted through an introducer needle such as needle 500 and into an intervertebral disc. FIG. 9 illustrates the delivery catheter of FIG. 8A extended through the introducer needle of FIG. 7C after the stylet 605 has been removed. The delivery catheter 800 includes an elongated hollow shaft 808 that has a straight portion and a preset bend 806, e.g., a curved portion at a distal end of the catheter. As shown in FIG. 8A, the hollow shaft 808 has an exposed length Lei and the catheter 800 an overall length of L_{C2}. In an example embodiment, Lei is about 8.1 inches (205.7 mm) and L_{C2} is about 9.3 inches (236.2 mm). A marking or indicator 813 on the proximal end 802, e.g., on the hub or handle 802, indicates to the operator the orientation of the bend 806. The marking 813 can be printed on the hub 802, as shown in FIG. 8B. The proximal and distal ends 802, 804 of the catheter 800 form openings that are in communication along the hollow shaft 808. The distal opening 812 (FIG. 9) is a side opening to the inside of the preset bend 806. The distal end of the delivery catheter includes a blunt tip.

As illustrated in FIG. 9, the delivery catheter 800 is longer than the introducer needle 500 and is extendable from the distal end of the cannula 508 of the introducer needle. In the example illustrated, an exposed length L_{N} of the introducer needle cannula 508 is about 6.0 inches (152.4 mm) and a length L_{C3} that the straight portion of the delivery catheter extends from the distal end of the hub 502 is about 6.3 inches (160.0 mm). As described herein, as the delivery catheter 800 is extended into a target tissue of an intervertebral disc, the combination of the blunt tip and preset bend allowing the delivery catheter to advance through but not out of an annulus fibrosus of the intervertebral disc.

The devices and methods described are improvements on prior approaches that use a double needle assembly to treat disc disease. Other approaches, for example, use needles to access single points on the annulus fibrosus by entering the nucleus pulposus and then traversing inner and outer walls of the annulus of the disc to access sites that are thought to be diseased or damaged. These needle delivery systems then deliver a therapeutic to the narrowly identified site. The shortcomings of these devices are that they do not treat the entire annulus fibrosus or substantially the entire circumference of the posterior annulus fibrosus. The current device addresses these and other shortcomings by assembling a novel combination of introducer needle and delivery catheter with specific preset bends and depth markings so that a clinician can reliably deliver therapy to inside a substantial portion of the circumference of the posterior annulus fibrosus of the disc space. The pre-set curve of the cannula can take on multiple different arches so that when the cannula is fully extended through the introducer cannula the shape of the cannula takes on an oval or elliptical shape and not a circular shape. An optional stylet can be assembled into the cannula to vary the persistence of length of the cannula. Various materials can be used to create the novel combination of depth of entry, curvature, persistence of length, to allow for delivery of a therapeutic through a catheter around a circular path within a boundary of tissue that resides subcutaneously, such as the annulus fibrosus. Different materials and combinations are described but are not meant to be limiting with respect to other such suitable materials that could be substituted.

In an embodiment, a minimally invasive intradiscal device is provided for delivering a therapeutic agent percutaneously to a target tissue. The device includes a rigid introducer needle with an elongated hollow rigid cannula having a first end and a second end, the first and second ends forming openings and in communication along the hollow shaft and a sharp stylet that protrudes from the distal end of the cannula, the shaft and stylet tip configured to allow insertion of the needle through the body and into a target tissue, the tip of the introducer needle can have a pre-specified bend or can be straight. A longer delivery catheter that fits coaxially through the introducer cannula when the stylet is removed, the delivery catheter having an elongated hollow cannula that also has a preset bend, the cannula having a first end and a second end, the first and second ends forming openings and in communication along the hollow shaft of the cannula and a blunt tip welded on the distal end of the cannula such that when the distal end of the delivery catheter exits the distal end of the introducer needle into the target tissue, the combination of the depth of the introducer needle into the tissue, the blunt tip catheter and preset bend allow the delivery catheter to be maneuvered through but not out of the target tissue. The blunt tip cap can be welded onto the distal end of the delivery cannula at an offset to improve the angle of attack to further facilitate the delivery catheter circumnavigating the inside of the target tissue. The target tissue can be, for example, the posterior outer annulus fibrosus. Nitinol is contemplated for the material of the delivery catheter. Stainless steel or Nitinol are contemplated for the welded tip. Alternatively, the cap can be from the Nitinol catheter by closing the end with a laser weld or other known means.

Other materials such as a polymer or a wire coil in the shape of a cannula are contemplated for the cannula of the delivery needle. The cannula of the delivery needle can have an open end and blunt stylet that protrudes past the end of the cannula. Additionally, the delivery cannula can be flexible without a preset bend and the curvature being provided by a nitinol stylet. The preset bent nitinol stylet is internally deployed in the delivery cannula, such a configuration causing the outer flexible cannula to take the shape of the internally deployed stylet. For example, a minimally invasive device for delivering a therapeutic agent subcutaneously comprised of a rigid introducer needle with an elongated hollow rigid cannula having a first end and a second end, the first and second ends forming openings and in communication along the hollow shaft and a sharp stylet that protrudes from the distal end of the introducer cannula, the shaft and stylet tip configured to allow insertion of the needle through the body and into a target tissue, and a longer delivery needle and cannula that fits coaxially through the introducer needle and cannula when the stylet is removed, the delivery needle has an elongated hollow flexible cannula formed from a wire coil or polyether ether ketone (PEEK), the coil or PEEK cannula having a first end and a second end, the first and second ends forming openings and in communication along the hollow shaft of the coil or PEEK cannula and a blunt stylet that protrudes from the distal end of the coil or PEEK cannula, the blunt tip stylet having a pre-set bend such that when the distal end of the delivery needle exits the distal end of the introducer needle into the target tissue, the combination of the internally deployed, preset bent blunt tip stylet allows the delivery cannula to advance in a circular trajectory to pass through but not out of the target tissue. The stylet is made of nitinol and can have a pre-set bend. Other material capable of maintaining a pre-set bend are also suitable. The internally deployed stylet inside the flexible coil or PEEK cannula causes the cannula to take on the same pre-set bend as the stylet. A closed end cap with at least one side port can be welded to the tip of the wire coil or PEEK cannula. The internally deployed stylet of the delivery cannula fits coaxially and dead ends against the cap on the distal end and can extend beyond the opening of the cannula on the proximal end. Pushing on the stylet advances the flexible wire or PEEK cannula forward. The combination of adjusting / setting the depth of the introducer needle along with the pre-set bend of the delivery cannula will allow the curved delivery needle to circumvent a large geography of the circular annular fibrosus of the disc without exiting into the nucleus pulposus or exiting outside the annular fibrosus. Any of the delivery cannulas disclosed can have a standard luer connection on the proximal end to allow for injection of the therapeutic as the cannula is withdrawn from the body.

Instead of PEEK, other suitable polymers may be used for the cannula.

As illustrated in FIGS. 3B, 3C and 4B, the opening 312 at the distal end of the catheter 300 can be a single hole oriented to the side and be can be part of the blunt end tip 304. The opening 312 is preferably oriented toward the center of the disc, or the nucleus pulposus. The fluid (e.g., therapeutic agent) injected through the catheter will flow toward the center of the disc in order to not place more pressure on tears or protrusions in the peripheral or outer part of the disc. An orientation marking on the catheter on the proximal handle end, e.g., hub 302, can be added so that the user can orient the port toward the inner portion of the disc. The marking can be a notch in the hub 302, a protrusion, or a symbol printed onto the hub.

In one embodiment not according to the invention, the delivery catheter does not have a pre-set bend, is highly flexible, and can have a blunt cap (tip) with a side port formed or welded onto the distal end. In this embodiment, a stylet provides the stiffness to advance the catheter, and has a pre-set curve, to allow the assembled stylet and catheter to travel through the annulus after passing coaxially through the introducer cannula. In this embodiment, the catheter is a slave to the bend of the stylet. The stylet can have varying degrees of stiffness based on changing the OD through centerless grinding the stylet. This can then change the persistence length of the assembled stylet and catheter as the assembly passes through the annulus after passing coaxially through the introducer cannula.

The stylet can be made from, for example, Nitinol. The catheter can be made from, for example, PEEK or a wire wound tube. The blunt cap with side port can be formed onto the end of the PEEK tube or a blunt cap with a side port can be made of steel and can be welded onto the end of the wire wound tube. Multiple different stylets having varying persistence length and curves can be placed in a kit. If one stylet does not have the appropriate rigidity to navigate the annulus fibrosus, it can be removed and a second stylet with either less or more rigidity can be inserted. In this embodiment, one size cannula can be used with multiple stylets available to customize the curve or stiffness of the cannula based on the particular needs of the individual patient. The different stylets can be switched out during the procure based on what the clinician encounters. The pre-set curve of the stylet can take on multiple different arches so that when the assembled catheter and stylet is fully extended through the introducer cannula the shape the catheter takes is an oval or elliptical shape and not a circular shape. The stylet can be removed leaving just the catheter in place. Fluids can be injected through the catheter as it is retrieved after removing the stylet.

It is contemplated that the introducer cannula can be made from various materials to include both polymers and metals including nitinol and that the introducer cannula can have a pre-set bend. It is contemplated that a stylet can be used for the injection catheter and introducer cannula in any of the embodiments and that the stylet for the injection catheter can have varying angles of bend along its length and varying OD so that a unique persistence of length can be established.

Embodiments of the invention provide an intervertebral disc apparatus that includes an introducer needle and cannula and a delivery catheter and cannula. The delivery catheter is at least partially positioned in the introducer needle. The delivery catheter is configured to be advanceable around the annulus within the inner and outer walls. A biologic (i.e. autologous platelet rich plasma, bone marrow aspirate, bone marrow concentrate, fibrin, or non-autologous fibrin or cells, or a combination) is contemplated to be injected through the delivery catheter. Energy such as laser energy delivered over a fiber is also contemplated to be delivered through the delivery catheter.

Embodiments include providing an externally guidable intervertebral disc apparatus, the disc having a nucleus pulposus, an annulus fibrosus, and an inner and outer wall of the annulus fibrosus, the nucleus pulposus having a first diameter and the surrounding annulus and nucleus pulposus having a second bigger diameter. Access to the annulus between the inner and outer walls being provided by an introducer needle and the delivery catheter being deployed between opposing sections of the inner and outer wall of the annulus. The delivery catheter comprises an internal lumen with an opening at a terminus, comprising a hollow shaft having a distal end and a proximal end having a longitudinal access, the hollow shaft being adapted to slide through the introducer lumen, the catheter having an intradiscal curved section at the distal end of the catheter, the intradiscal curved section being extendable through the opening of the introducer and having sufficient rigidity to be advanceable through the tissue of the annulus of the disc between the inner and outer wall of the annulus fibrosus. Under a force applied longitudinally to the proximal end of the delivery catheter, such catheter under force having a bend and insufficient penetration ability to be advanceable through the wall of the outer annulus fibrosus but sufficient curvature and persistence of length to travel through the annulus fibrosus. The delivery catheter has depth markings on the proximal end. The delivery cannula is retracted from the disc in set intervals based on the depth markings. At each interval, a therapeutic agent is delivered to the disc, allowing for delivery of said agent broadly along the entire posterior circumference of the disc. Such agents contemplated can be platelet rich plasma, bone marrow aspirate or concentrate, adipose derived cells, fibrin autologous or non-autologous, laser energy delivered over a fiber optic that coaxially fits through the delivery catheter or other types of energy delivered through a probe that coaxially fits through the delivery catheter. The depth markings on the delivery cannula can be for instance every half centimeter.

A particular benefit of the device is that it allows for delivery of the biologic from both a posterior and anterior angle of attack when delivering a biologic or fluids to a disc.

The following is a step by step description of how the device can be used in practice.

The procedure is to be performed in an outpatient surgical suite or ambulatory care center under strict sterile conditions. It is to be performed under fluoroscopic guidance with intravenous sedation. The disc levels treated will be determined pre-procedure by the patient's history, physical examination, pain drawing and radiological testing.

The patient is placed in a prone position on the fluoroscopy table. After the usual sterile skin preparation, sterile draping of the treatment area, and local anesthesia a 17-gauge introducer needle is place using the standard extra-pedicular approach into the anterior lateral annulus fibrosus. Both anterior-posterior and lateral fluoroscopic images confirm proper needle placement. The stylet of the introducer needle is removed and then the semi-rigid catheter is manually advanced under fluoroscopic guidance into the anterior annulus fibrosus using the preset curved blunt tip to navigate. The catheter is slowly and gently advanced parallel to the end plates under lateral fluoroscopic imaging to the contralateral anterior annulus fibrosus and then to the contralateral posterior annulus fibrosus, across the posterior central annulus fibrosus, and finally to the ipsilateral posterior annulus fibrosus. Proper catheter placement is confirmed by both anterior-posterior and lateral fluoroscopic images.

The desired fluid is then injected slowly while the catheter is withdrawn in predictable increments to deliver the fluid throughout the whole posterior outer annulus fibrosus. Typical injected volumes would be 0.5-1.0 cc of fluid every 0.5 - 1.0 cm of movement. During this part of the procedure the side port is directed toward the inner disc as to not put abnormal force on the outer annulus fibrosus in order to not worsen the disc protrusion. A marker on the end of the disc catheter will notify the interventionalist as to the direction of the side port. Once the entire posterior annulus fibrosus has been bathed in fluid, the catheter is withdrawn back through the introducer needle and both the introducer needle and the catheter are removed manually from the disc. A sterile dressing is the applied to the wound and the patient is taken to the post-procedure recovery area to monitor for any adverse events.

While example embodiments have been particularly shown and described, it will be understood by those skilled in the art that various changes in form and details may be made therein without departing from the scope of the embodiments encompassed by the invention, which is defined solely by the appended claims.

## Claims

1. A minimally invasive device for delivering a therapeutic agent percutaneously to a target tissue of an intervertebral disc, the device comprising:
a rigid introducer needle (200, 500) including an elongated cannula (208, 508) having a proximal end and a distal end, the proximal and distal ends forming openings and being in communication along the cannula, and a stylet (205, 605) extending through the cannula and having a tip that closes the opening at the distal end of the cannula, the cannula and stylet configured to allow insertion of the needle percutaneously into tissue; and a delivery catheter (300, 800) that fits coaxially through the introducer needle when the stylet is removed, the delivery catheter being longer than the introducer needle and including an elongated hollow shaft (308, 808), the delivery catheter having a proximal end and a distal end, the proximal and distal ends forming openings and being in communication along the hollow shaft, the delivery catheter being extendable from the distal end of the introducer needle and into a target tissue of an intervertebral disc;
the device **characterized in that** the elongated hollow shaft has a preset bend (306, 806) providing a pre-shaped curved distal portion along a length of the delivery catheter, wherein the preset bend (306, 806) of the delivery catheter is such that the catheter upon exiting the introducer needle follows an oval path having an initial diameter between about 1.5 cm and about 3.5 cm, the distal opening being a side opening (312, 812) to the inside of the preset bend, the distal end of the delivery catheter including a closed, blunt tip (304, 804), the combination of the blunt tip and preset bend allowing the delivery catheter to advance through but not out of an annulus fibrosus of the intervertebral disc, the side opening being orientable toward a center of the intervertebral disc for delivering a therapeutic agent to the target tissue.

2. The device according to claim 1, wherein the blunt tip (304, 804) is positioned on the hollow shaft (308, 808) at the distal end of the delivery catheter at an angle that is offset from the center line of the delivery catheter.

3. The device according to claim 2, wherein the blunt tip (304, 804) is welded on the hollow shaft (308, 808) at the distal end of the delivery catheter and made of a material different from the shaft.

4. The device according to claim 3, wherein the hollow shaft (308, 808) of the delivery catheter is made of nitinol, and wherein optionally the blunt tip (304, 804) of the delivery catheter is made of stainless steel.

5. The device according to claim 1, wherein the cannula (208, 508) of the introducer needle has a preset bend (206, 506) at the distal end of the cannula.

6. The device according to claim 5, wherein the preset bend at the distal end of the cannula is about 3 mm to about 9 mm from the distal tip with an angle between about 4 degrees and about 6 degrees, preferably about 6.25 mm from the distal tip with an angle of about 5 degrees.

7. The device according to any one of claims 1 to 6, wherein the introducer needle (200, 500) has a two-part handle with a first part of the handle attached to the cannula (208, 508) and a second part of the handle attached to the stylet (205, 605), each part attached at the proximal end of the introducer needle, the second part of the handle and stylet being selectively removable from the cannula, and wherein the first part of the handle defines a passageway in communication with the opening at the distal end of the introducer needle when the first part of the handle and stylet are removed.

8. The device according to claim 7, wherein the first part of the handle includes a luer hub (202, 502).

9. The device according to any one of claims 1 to 8, wherein the delivery catheter (300, 800) includes a luer lock connection at the proximal end of the delivery catheter, and wherein optionally the luer lock enables delivery of the therapeutic agent from a syringe holding the therapeutic agent and being coupled to the luer lock.

10. The device according to any one of claims 1 to 9, wherein the introducer needle (200, 500) has markings on the outside that allow determining the distance of insertion into the target tissue.

11. The device according to claim 10, wherein the delivery catheter (300, 800) has one or more markings on the proximal end on the outside that allow determining the distance the delivery catheter is inserted beyond the introducer needle or retracted from the target tissue.

12. The device according to any one of claims 1 to 11, wherein the target tissue is a posterior outer third of the annulus fibrosus of the intervertebral disc.

13. The device according to any one of claims 1 to 12, wherein the introducer needle (200, 500) includes markings (207, 507) that facilitate monitoring of depth of entry into tissue, the introducer needle configured to be inserted a pre-determined depth into the annulus fibrosus so that the combination of the starting point where the distal end of the delivery catheter exits the distal end of the introducer needle, the length the delivery catheter extends beyond the introducer needle when the catheter is fully extended beyond the distal end of the introducer needle, the preset bend of the delivery catheter, the blunt tip on the end of the delivery catheter, and stiffness or persistence length of the delivery catheter, results in the delivery catheter exiting into and staying within the tissue of the annulus fibrosus.

14. The device according to any one of claims 1 to 13, wherein the delivery catheter (300, 800) is configured to be retracted from the target tissue in set intervals, at each interval, a portion of a therapeutic agent being introduced into the annulus fibrosus, and wherein optionally the therapeutic agent is platelet rich plasma, bone marrow aspirate, bone marrow concentrate, adipose-derived cells, autologous or non-autologous fibrin, medications, contrast dye, or energy delivered over a fiber or probe.

15. The device according to any one of claims 1 to 14, wherein the length that the delivery catheter (300, 800) is extendable past the distal end of the introducer needle (200, 500) is sufficient to travel at least 75% of the circumference of a circle based on the preset bend of the delivery catheter.

## Patentansprüche

1. Minimal-invasive Vorrichtung zum perkutanen Zuführen eines Therapeutikums zu einem Zielgewebe einer Bandscheibe, wobei die Vorrichtung Folgendes umfasst:
eine starre Einführnadel (200, 500), die eine längliche Kanüle (208, 508) mit einem proximalen Ende und einem distalen Ende aufweist, wobei das proximale und das distale Ende Öffnungen bilden und entlang der Kanüle in Verbindung stehen, und ein Stilett (205, 605), das sich durch die Kanüle erstreckt und eine Spitze aufweist, die die Öffnung an dem distalen Ende der Kanüle verschließt, wobei die Kanüle und das Stilett dazu ausgestaltet sind, ein perkutanes Einführen der Nadel in Gewebe zu gestatten, und einen Zuführungskatheter (300, 800) der koaxial durch die Einführnadel passt, wenn das Stilett entfernt wird, wobei der Zuführungskatheter länger als die Einführnadel ist und einen länglichen hohlen Schaft (308, 808) aufweist, wobei der Zuführungskatheter ein proximales Ende und ein distales Ende aufweist, wobei das proximale und das distale Ende Öffnungen bilden und entlang des hohlen Schafts in Verbindung stehen, wobei der Zuführungskatheter von dem distalen Ende der Einführnadel und in ein Zielgewebe einer Bandscheibe ausfahrbar ist,
wobei die Vorrichtung **dadurch gekennzeichnet ist, dass** der längliche hohle Schaft eine voreingestellte Biegung (306, 806) aufweist, die einen vorgeformten gekrümmten distalen Abschnitt entlang einer Länge des Zuführungskatheters bereitstellt, wobei die voreingestellte Biegung (306, 806) des Zuführungskatheters derart ist, dass der Katheter beim Austritt aus der Einführnadel einer ovalen Bahn mit einem Anfangsdurchmesser zwischen etwa 1,5 cm und etwa 3,5 cm folgt, wobei die distale Öffnung eine Seitenöffnung (312, 812) zu dem Inneren der voreingestellten Biegung ist, wobei das distale Ende des Zuführungskatheters eine geschlossene, stumpfe Spitze (304, 804) aufweist, wobei die Kombination aus der stumpfen Spitze und der voreingestellten Biegung es dem Zuführungskatheter gestattet, durch einen Anulus fibrosus der Bandscheibe vorzurücken, aber nicht aus diesem heraus, wobei die Seitenöffnung zu einer Mitte der Bandscheibe hin ausgerichtet werden kann, um dem Zielgewebe ein Therapeutikum zuzuführen.

2. Vorrichtung nach Anspruch 1, wobei die stumpfe Spitze (304, 804) an dem hohlen Schaft (308, 808) an dem distalen Ende des Zuführungskatheters in einem Winkel positioniert ist, der von der Mittellinie des Zuführungskatheters versetzt ist.

3. Vorrichtung nach Anspruch 2, wobei die stumpfe Spitze (304, 804) an dem distalen Ende des Zuführungskatheters an dem hohlen Schaft (308, 808) angeschweißt ist und aus einem von dem Schaft verschiedenen Material hergestellt ist.

4. Vorrichtung nach Anspruch 3, wobei der hohle Schaft (308, 808) des Zuführungskatheters aus Nitinol hergestellt ist und wobei optional die stumpfe Spitze (304, 804) des Zuführungskatheters aus Edelstahl hergestellt ist.

5. Vorrichtung nach Anspruch 1, wobei die Kanüle (208, 508) der Einführnadel eine voreingestellte Biegung (206, 506) an dem distalen Ende der Kanüle aufweist.

6. Vorrichtung nach Anspruch 5, wobei sich die voreingestellte Biegung an dem distalen Ende der Kanüle etwa 3 mm bis etwa 9 mm von der distalen Spitze mit einem Winkel zwischen etwa 4 Grad und etwa 6 Grad, vorzugsweise etwa 6,25 mm von der distalen Spitze mit einem Winkel von etwa 5 Grad, befindet.

7. Vorrichtung nach einem der Ansprüche 1 bis 6, wobei die Einführnadel (200, 500) einen zweiteiligen Griff aufweist, wobei ein erster Teil des Griffs an der Kanüle (208, 508) angebracht ist und ein zweiter Teil des Griffs an dem Stilett (205, 605) angebracht ist, wobei jeder Teil an dem proximalen Ende der Einführnadel angebracht ist, wobei der zweite Teil des Griffs und das Stilett gezielt von der Kanüle entfernbar sind, und wobei der erste Teil des Griffs einen Durchgang definiert, der mit der Öffnung an dem distalen Ende der Einführnadel in Verbindung steht, wenn der erste Teil des Griffs und das Stilett entfernt werden.

8. Vorrichtung nach Anspruch 7, wobei der erste Teil des Griffs einen Luer-Ansatz (202, 502) aufweist.

9. Vorrichtung nach einem der Ansprüche 1 bis 8, wobei der Zuführungskatheter (300, 800) eine Luer-Lock-Verbindung an dem proximalen Ende des Zuführungskatheters aufweist und wobei optional der Luer-Lock das Zuführen des Therapeutikums von einer Spritze ermöglicht, die das Therapeutikum enthält und an den Luer-Lock gekoppelt ist.

10. Vorrichtung nach einem der Ansprüche 1 bis 9, wobei die Einführnadel (200, 500) Markierungen an der Außenseite aufweist, die das Bestimmen der Einführungsdistanz in das Zielgewebe ermöglichen.

11. Vorrichtung nach Anspruch 10, wobei der Zuführungskatheter (300, 800) eine oder mehrere Markierungen an dem proximalen Ende an der Außenseite aufweist, dank derer die Distanz, über die der Zuführungskatheter über die Einführnadel hinaus eingeführt oder aus dem Zielgewebe zurückgezogen wird, bestimmt werden kann.

12. Vorrichtung nach einem der Ansprüche 1 bis 11, wobei das Zielgewebe ein hinteres äußeres Drittel des Anulus fibrosus der Bandscheibe ist.

13. Vorrichtung nach einem der Ansprüche 1 bis 12, wobei die Einführnadel (200, 500) Markierungen (207, 507) aufweist, die die Überwachung der Eindringtiefe in Gewebe erleichtern, wobei die Einführnadel dazu ausgestaltet ist, eine vorbestimmte Tiefe in den Anulus fibrosus eingeführt zu werden, so dass die Kombination des Startpunkts, an dem das distale Ende des Zuführungskatheters aus dem distalen Ende der Einführnadel austritt, der Länge, über die sich der Zuführungskatheter über die Einführnadel hinaus erstreckt, wenn der Katheter vollständig über das distale Ende der Einführnadel hinaus ausgefahren ist, der voreingestellten Biegung des Zuführungskatheters, der stumpfen Spitze an dem Ende des Zuführungskatheters und der Steifigkeit oder Persistenzlänge des Zuführungskatheters dazu führt, dass der Zuführungskatheter in das Gewebe des Anulus fibrosus austritt und darin verbleibt.

14. Vorrichtung nach einem der Ansprüche 1 bis 13, wobei der Zuführungskatheter (300, 800) dazu ausgestaltet ist, in festgelegten Intervallen aus dem Zielgewebe zurückgezogen zu werden, wobei in jedem Intervall ein Teil eines Therapeutikums in den Anulus fibrosus eingeführt wird, und wobei optional das Therapeutikum plättchenreiches Plasma, Knochenmarkaspirat, Knochenmarkkonzentrat, von Fettgewebe stammende Zellen, autologes oder nicht-autologes Fibrin, Medikamente, Kontrastmittel oder Energie, die über eine Faser oder eine Sonde abgegeben wird, ist.

15. Vorrichtung nach einem der Ansprüche 1 bis 14, wobei die Länge, über die der Zuführungskatheter (300, 800) über das distale Ende der Einführnadel (200, 500) hinaus ausfahrbar ist, ausreichend ist, so dass er sich, basierend auf der voreingestellten Biegung des Zuführungskatheters, über mindestens 75 % des Umfangs eines Kreises bewegt.

## Revendications

1. Dispositif mini-invasif pour administrer un agent thérapeutique de façon percutanée à un tissu cible d'un disque intervertébral, le dispositif comprenant :
une aiguille d'introduction rigide (200, 500) comportant une canule allongée (208, 508) ayant une extrémité proximale et une extrémité distale, les extrémités proximale et distale formant des ouvertures et étant en communication le long de la canule, et un stylet (205, 605) s'étendant à travers la canule et ayant une pointe qui ferme l'ouverture au niveau de l'extrémité distale de la canule, la canule et le stylet étant conçus pour permettre l'insertion de l'aiguille de façon percutanée dans un tissu ; et un cathéter d'administration (300, 800) qui s'ajuste de manière coaxiale à travers l'aiguille d'introduction lorsque le stylet est retiré, le cathéter d'administration étant plus long que l'aiguille d'introduction et comportant un arbre creux allongé (308, 808), le cathéter d'administration ayant une extrémité proximale et une extrémité distale, les extrémités proximale et distale formant des ouvertures et étant en communication le long de l'arbre creux, le cathéter d'administration pouvant s'étendre à partir de l'extrémité distale de l'aiguille d'introduction et jusque dans un tissu cible d'un disque intervertébral ;
le dispositif étant **caractérisé en ce que** l'arbre creux allongé a une courbure prédéfinie (306, 806) fournissant une partie distale incurvée préformée sur une longueur du cathéter d'administration, la courbure prédéfinie (306, 806) du cathéter d'administration étant telle que le cathéter, à sa sortie de l'aiguille d'introduction, suit une trajectoire ovale ayant un diamètre initial compris entre environ 1,5 cm et environ 3,5 cm, l'ouverture distale étant une ouverture latérale (312, 812) vers l'intérieur de la courbure prédéfinie, l'extrémité distale du cathéter d'administration comportant une pointe émoussée fermée (304, 804), la combinaison de la pointe émoussée et de la courbure prédéfinie permettant au cathéter d'administration d'avancer à travers, mais pas hors, d'un anneau fibreux du disque intervertébral, l'ouverture latérale pouvant être orientée vers un centre du disque intervertébral pour l'administration d'un agent thérapeutique au tissu cible.

2. Dispositif selon la revendication 1, la pointe émoussée (304, 804) étant positionnée sur l'arbre creux (308, 808) au niveau de l'extrémité distale du cathéter d'administration selon un angle qui est décalé de la ligne centrale du cathéter d'administration.

3. Dispositif selon la revendication 2, la pointe émoussée (304, 804) étant soudée sur l'arbre creux (308, 808) au niveau de l'extrémité distale du cathéter d'administration et étant composée d'un matériau différent de l'arbre.

4. Dispositif selon la revendication 3, l'arbre creux (308, 808) du cathéter d'administration étant composé de nitinol, et éventuellement la pointe émoussée (304, 804) du cathéter d'administration étant composée d'acier inoxydable.

5. Dispositif selon la revendication 1, la canule (208, 508) de l'aiguille d'introduction ayant une courbure prédéfinie (206, 506) au niveau de l'extrémité distale de la canule.

6. Dispositif selon la revendication 5, la courbure prédéfinie au niveau de l'extrémité distale de la canule étant à environ 3 mm à environ 9 mm de la pointe distale avec un angle compris entre environ 4 degrés et environ 6 degrés, de préférence à environ 6,25 mm de la pointe distale avec un angle d'environ 5 degrés.

7. Dispositif selon l'une quelconque des revendications 1 à 6, l'aiguille d'introduction (200, 500) ayant une poignée en deux parties avec un première partie de la poignée fixée à la canule (208, 508) et une deuxième partie de la poignée fixée au stylet (205, 605), chaque partie étant fixée au niveau de l'extrémité proximale de l'aiguille d'introduction, la deuxième partie de la poignée et le stylet étant amovibles de façon sélective de la canule, et la première partie de la poignée définissant un passage en communication avec l'ouverture au niveau de l'extrémité distale de l'aiguille d'introduction lorsque la première partie de la poignée et le stylet sont retirés.

8. Dispositif selon la revendication 7, la première partie de la poignée comportant un embout Luer (202, 502) .

9. Dispositif selon l'une quelconque des revendications 1 à 8, le cathéter d'administration (300, 800) comportant un raccord Luer-Lock au niveau de l'extrémité proximale du cathéter d'administration, et éventuellement le Luer-Lock permettant l'administration de l'agent thérapeutique à partir d'une seringue contenant l'agent thérapeutique et accouplée au Luer-Lock.

10. Dispositif selon l'une quelconque des revendications 1 à 9, l'aiguille d'introduction (200, 500) ayant des repères sur l'extérieur qui permettent de déterminer la distance d'insertion dans le tissu cible.

11. Dispositif selon la revendication 10, le cathéter d'administration (300, 800) ayant un ou plusieurs repères sur l'extrémité proximale sur l'extérieur qui permettent de déterminer la distance à laquelle le cathéter d'administration est inséré au-delà de l'aiguille d'introduction ou rétracté du tissu cible.

12. Dispositif selon l'une quelconque des revendications 1 à 11, le tissu cible étant un tiers externe postérieur de l'anneau fibreux du disque intervertébral.

13. Dispositif selon l'une quelconque des revendications 1 à 12, l'aiguille d'introduction (200, 500) comportant des repères (207, 507) qui facilitent la surveillance de la profondeur d'entrée dans le tissu, l'aiguille d'introduction étant conçue pour être insérée à une profondeur prédéterminée dans l'anneau fibreux de sorte que la combinaison du point de départ où l'extrémité distale du cathéter d'administration sort de l'extrémité distale de l'aiguille d'introduction, de la longueur du cathéter d'administration qui s'étend au-delà de l'aiguille d'introduction lorsque le cathéter est complètement étendu au-delà de l'extrémité distale de l'aiguille d'introduction, de la courbure prédéfinie du cathéter d'administration, de la pointe émoussée sur l'extrémité du cathéter d'administration et de la rigidité ou de la longueur de persistance du cathéter d'administration, aboutisse à ce que le cathéter d'administration sorte dans le tissu de l'anneau fibreux et y reste.

14. Dispositif selon l'une quelconque des revendications 1 à 13, le cathéter d'administration (300, 800) étant conçu pour être rétracté du tissu cible à intervalles définis, à chaque intervalle, une partie d'un agent thérapeutique étant introduite dans l'anneau fibreux, et éventuellement l'agent thérapeutique étant un plasma riche en plaquettes, un aspirat de moelle osseuse, un concentré de moelle osseuse, des cellules dérivées de tissu adipeux, de la fibrine autologue ou non autologue, des médicaments, un produit de contraste, ou de l'énergie délivrée par une fibre ou une sonde.

15. Dispositif selon l'une quelconque des revendications 1 à 14, la longueur à laquelle le cathéter d'administration (300, 800) peut s'étendre au-delà de l'extrémité distale de l'aiguille d'introduction (200, 500) étant suffisamment grande pour parcourir au moins 75 % de la circonférence d'un cercle sur la base de la courbure prédéfinie du cathéter d'administration.
